# FASCICULE DE BREVET EUROPEEN

(11) **EP 0 621 793 B1**
(45) Date de publication et mention de la délivrance du brevet: **28.08.1996**
(21) Numéro de dépôt: 93904082.0
(22) Date de dépôt: 14.01.1993
(51) Int. Cl.: A61M 29/02

(54) **CATHETER DE DILATATION**
DILATATIONSKATHETER
DILATING CATHETER

(30) Priorité: 17.01.1992 FR 9200499
(43) Date de publication de la demande: 02.11.1994
(73) Titulaire: LABORATOIRES NYCOMED S.A., F-75644 Paris Cédex 13 (FR)
(72) Inventeur: BOUSSIGNAC, Georges, F-92160 Antony (FR); LABRUNE, Jean-Claude, F-92100 Boulogne (FR)
(74) Mandataire: Hubert, Philippe
(86) Numéro de dépôt international: FR9300036
(87) Numéro de publication internationale: WO9313826

(56) Documents cités:
- EP-A- 0 418 147
- US-A- 4 581 017
- US-A- 4 646 742
- US-A- 4 790 315
- US-A- 4 877 031

## Description

La présente invention a pour objet un cathéter de dilatation à perfusion destiné à être introduit dans un canal corporel tel que notamment un vaisseau sanguin.

L'invention trouve principalement application dans le domaine du traitement des affections des artères coronaires mais peut être également utilisée dans le domaine du traitement des affections d'autres canaux corporels, comme par exemple l'oesophage ou l'urètre.

Ces affections résultent généralement de la présence, sur les parois internes du canal de dépôts entraînant des rétrécissements ou sténoses de celui-ci.

Dans le traitement de ces affections, un cathéter de dilation est généralement utilisé :
- d'une part, pour rétablir la section normale de passage du canal au niveau de la sténose par compression à l'aide d'un ballon (ou ballonet),
- d'autre part, pour injecter, par exemple à travers la paroi du ballon un produit de traitement destiné à rendre durable le rétablissement de la section de passage du canal en formant éventuellement in-situ une prothèse de section correspondante.

Lors de la compression (écrasement des dépôts par le ballon), le canal est généralement complètement obturé par le ballon. Or, il est nécessaire de maintenir, pendant l'intervention, une circulation minimale de fluide dans le canal corporel, afin de ne pas risquer d'endommager les tissus ou organes situés en aval du ballon.

Diverses solutions ont été proposées dans l'état de la technique pour résoudre ce problème.

Ainsi, dans les documents US 4 771 777, 4 790 315 ou 4 892 519, il est proposé de faire circuler le fluide corporel au travers de l'organe tubulaire interne du cathéter de dilatation servant au passage du guide.

Cette solution n'est cependant pas satisfaisante car la section de passage du fluide corporel est limitée, d'une part, en raison du faible diamètre de l'organe tubulaire interne, d'autre part, en raison de la présence du guide.

Dans le document US 5,000,734, il est proposé de faire circuler le fluide corporel au travers du ballon. A cet effet, le ballon comporte, à l'état gonflé, une pluralité de conduits longitudinaux.

Cette solution qui constitue un perfectionnement relativement à celle décrite dans les documents précités n'est cependant pas entièrement satisfaisante.

En effet, comme on le comprend, la section de passage du fluide corporel dépend du nombre et du diamètre des conduits prévus sur le ballon.

Or, de tels conduits sont relativement difficiles à réaliser et un nombre trop important de conduits nuirait à la résistance mécanique du ballon.

Dans ces conditions, la présente invention a pour but de résoudre le problème technique consistant en la fourniture d'un cathéter de dilatation d'une nouvelle conception, assurant une section de passage du fluide corporel importante, qui puisse être réalise facilement à l'échelle industrielle, et dont la mise en oeuvre reste relativement aisée.

La solution, conforme à la présente invention, pour résoudre ce problème technique consiste en un cathéter de dilatation, destiné à être introduit dans un canal corporel tel que notamment un vaisseau sanguin, du type comprenant :
- un élément tubulaire externe comportant une partie distale et une partie proximale, présentant au niveau de sa partie distale une portion déformable radialement comportant un ballon, et de part et d'autre de ce ballon au moins un orifice communiquant avec ledit canal corporel et permettant le passage du fluide circulant dans le canal corporel à l'intérieur dudit élément tubulaire externe ;
- un élément tubulaire interne, disposé intérieurement audit élément tubulaire externe, et permettant le passage d'un guide ;
- un micro-tube, disposé de préférence intérieurement audit organe tubulaire externe, débouchant à une extrémité à l'intérieur dudit ballon et relié à son autre extrémité à une source d'alimentation en fluide pour permettre le gonflage et le dégonflage dudit ballon;
- ledit ballon comprenant une paroi externe destinée à venir en contact avec la paroi interne dudit canal corporel, en position d'utilisation caractérisé en ce que : une paroi interne est fixée de manière étanche à ladite paroi externe et est susceptible de délimiter avec celle-ci, en position d'utilisation, une cavité interne annulaire ;
- et en ce qu'il comprend en outre un élément de support conformé pour maintenir en position d'utilisation ladite paroi interne du ballon, tout en permettant la circulation du fluide corporel dans l'espace compris entre ledit élément interne et la paroi interne du ballon.

Ainsi, l'originalité de la présente invention vis-à-vis de l'état de la technique réside dans le fait qu'en utilisant un ballon à double paroi, associé à un moyen de support approprié, on libère une partie importante de la section du cathéter comprise entre la paroi interne du ballon et l'élément tubulaire interne servant au passage du guide, en permettant ainsi de maintenir une circulation quasiment normale du fluide dans le canal corporel.

Un tel cathéter peut donc être maintenu en position d'utilisation dans le canal corporel pendant une durée de temps très importante tout à fait suffisante pour rendre durable le rétablissement de la section normale de passage du canal par des traitements appropriés connus dans la technique et rappelés précédemment.

L'expression "position d'utilisation" utilisée dans le cadre de la présente description et des revendications désigne la position de traitement de la sténose, dans laquelle le ballon est à l'état gonflé.

L'invention sera mieux comprise et d'autres buts, caractéristiques et avantages de celle-ci apparaîtront plus clairement à la lecture de la description explicative qui va suivre, faite en référence aux dessins schématiques annexés donnés uniquement à titre d'exemples non-limitatifs illustrant deux modes de réalisation actuellement préférés de l'invention et dans lesquels :
- la figure 1 est une vue en coupe longitudinale partielle d'un cathéter de dilatation conforme à un premier mode de réalisation de l'invention avant gonflage du ballon ;
- la figure 2 est une vue semblable à la figure 1 du même cathéter, pendant la dilatation de la sténose ;
- la figure 3 est une vue semblable à la figure 1 du même cathéter de dilatation montrant le maintien du gonflage et le libre passage du fluide corporel ;
- la figure 4 est une vue en coupe transversale selon la ligne IV-IV de la figure 2 ;
- la figure 5 est une vue en coupe transversale selon la ligne V-V de la figure 3 prise au niveau du ballon ;
- la figure 6 est une vue en coupe transversale selon la ligne VI-VI de la figure 3 prise en amont du ballon ;
- la figure 7 est une vue en coupe transversale selon la ligne VII-VII de la figure 3 prise au niveau de la partie proximale du cathéter
- la figure 8 est une vue en coupe longitudinale partielle d'un cathéter de dilatation conforme à un second mode de réalisation de l'invention, avant gonflage du ballon ;
- la figure 9 est une vue semblable à la figure 8 du même cathéter pendant la dilatation de la sténose ; et
- la figure 10 est une vue semblable à la figure 9 du même cathéter montrant le maintien par un élément formant ressort du gonflage avec libre passage du fluide corporel.

Dans la description qui va suivre, le canal corporel choisi à titre d'exemple est un vaisseau sanguin non représenté comme en particulier une artère coronaire.

En outre, on désignera par le terme "avant" la partie distale d'un élément et par le terme "arrière" sa partie proximale.

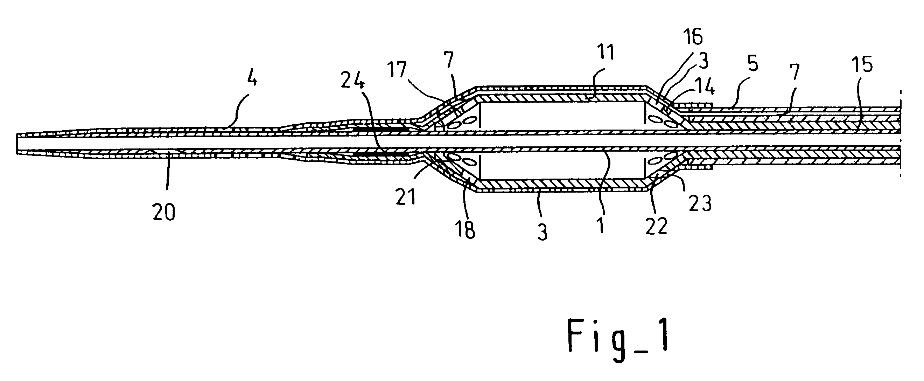

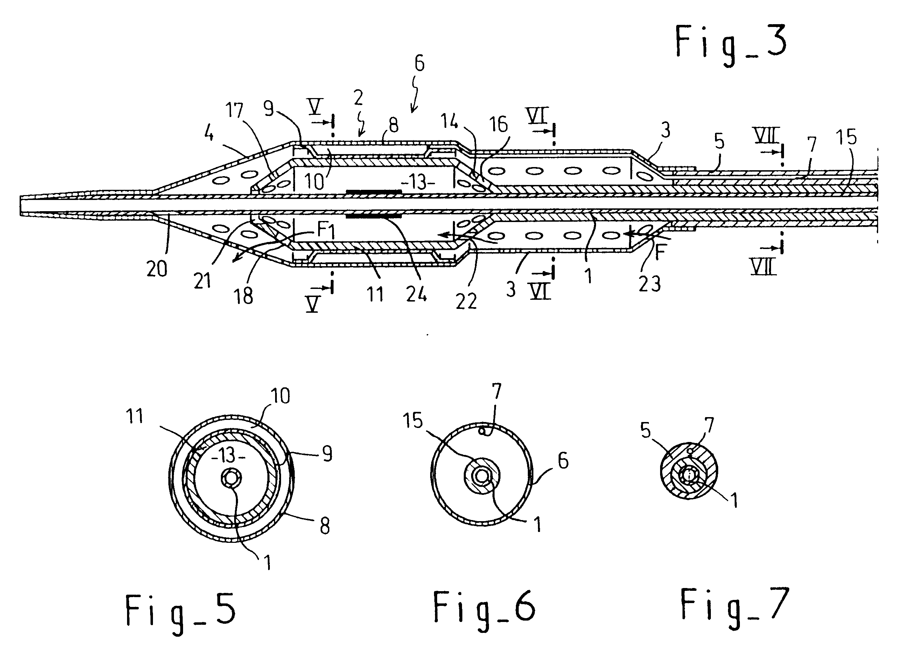

On a donc représenté aux figures 1 à 7 la partie distale d'un cathéter de dilatation conforme à un premier mode de réalisation de l'invention. Ce cathéter comporte un élément tubulaire externe comportant une partie distale et une partie proximale non représentée et un élément tubulaire interne 1 disposé intérieurement audit élément tubulaire externe, de façon concentrique ou excentrique (voir figure 7).

L'élément tubulaire interne 1 présente une section transversale sensiblement circulaire et permet le passage d'un guide généralement réalisé sous forme d'un fil. Ce guide est utilisé pour faciliter le placement du cathéter de dilatation au delà de l'extrémité distale du cathéter guide non représenté.

L'élément tubulaire interne 1 peut être réalisé par exemple en une matière telle qu'un thermoplastique semi-rigide comme par exemple un polyéthylène, un polyamide, un polyuréthane ou un copolymère du type Pebax ® commercialisé par la Société Atochem.

L'élément tubulaire externe présente au niveau de sa partie distale une portion déformable radialement et comportant un ballon gonflable 2, et de part et d'autre de ce ballon au moins un orifice 3 (en amont) et 4 (en aval) communiquant en position d'utilisation avec le vaisseau sanguin dans lequel le cathéter est introduit, en permettant ainsi le passage du sang circulant dans le vaisseau à l'intérieur de l'élément tubulaire externe.

Dans l'exemple représenté aux figures, l'élément tubulaire externe comprend un tube flexible 5 sur la partie avant de laquelle est fixée de façon étanche, par exemple par thermosoudure ou au moyen d'un adhésif, une partie formant gaine déformable radialement, généralement référencée 6, faisant corps avec le ballon 2. Le ballon 2 peut être intégré à la gaine 6, comme dans l'exemple représenté, ou rapporté sur celle-ci.

Dans le mode de réalisation représenté, la partie formant gaine 6 est fixée de façon étanche par sa partie avant de forme générale cylindro- conique en position d'utilisation, à l'élément tutulaire interne 1 dont la partie avant se prolonge au delà de l'extrémité avant du tube externe 5.

Dans une variante de réalisation, la partie formant gaine 6 peut être intégrée, c'est-à-dire réalisée en une seule pièce avec l'élément tubulaire externe. Dans ce cas, ce dernier aura une longueur sensiblement égale à celle de l'élément tubulaire interne 1.

Le tube 5 pourra être réalisé par exemple en une matière telle que celle servant à réaliser l'élément tubulaire interne 1.

Le ballon 2 peut être réalisé en une matière telle que un polyéthylène réticulé, un polyamide ou un polyéthylène téréphtalate biorientés. Ces matières ont été décrites dans l'état de la technique.

La gaine déformable 6 peut être réalisée dans la même matière que le ballon 2 ou encore dans une matière compatible avec celle-ci.

L'élément tubulaire externe comporte un micro-canal ou micro-tube 7 sensiblement longitudinal, débouchant à son extrémité avant à l'intérieur du ballon 2 et relié à son autre extrémité à une source d'alimentation en fluide non représentée, permettant le gonflage et le dégonflage du ballon 2.

Le micro-canal ou micro-tube 7 peut être réalisé par exemple en une matière telle que celle servant à réaliser le tube 5.

Le ballon 2 comprend une paroi externe 8 de forme généralement cylindrique en position d'utilisation, destinée à venir en contact avec la paroi interne du vaisseau en écrasant les dépôts entraînant la sténose. Le ballon 2 comporte en outre une paroi interne 9 fixée latéralement de manière étanche à la paroi externe 8 et délimitant avec celle-ci une cavité interne 10.

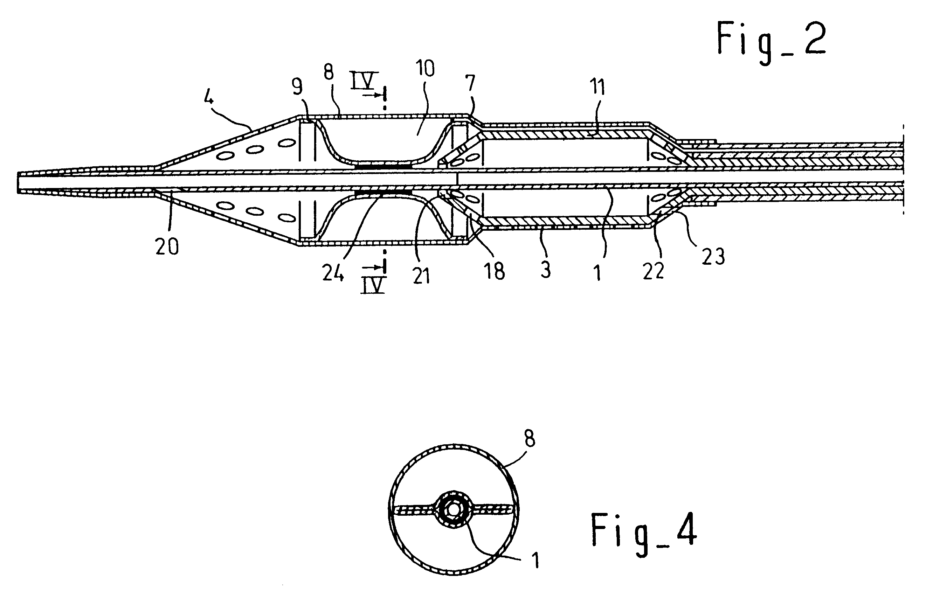

Lorsque le ballon 2 est gonflé, la paroi interne 9 se collabe sous l'effet de la pression (voir figures 2 et 4).

On peut ainsi réaliser la dilatation de la sténose de façon classique.

Il est également possible, et ceci constitue le fondement de la présente invention, de donner à la cavité interne 10 une forme annulaire à section transversale sensiblement rectangulaire, en prévoyant un élément de support de la paroi interne 9 destiné à reprendre les efforts dûs à la pression à l'intérieur du ballon, et à libérer une large lumière pour le passage du fluide corporel.

Dans ce premier mode de réalisation de l'invention, l'élément de support précité comprend un manchon 11 de forme généralement cylindrique, et dont la longueur est sensiblement égale à celle du ballon 2.

Le manchon 11 est déplaçable longitudinalement entre une position de repos, dans laquelle il se trouve en amont du ballon 2 (figure 2) et une position de travail dans laquelle il vient supporter et maintenir la paroi interne 9 du ballon 2 (figure 3) en l'écartant de l'élément tubulaire 1 et en créent ainsi une lumière 13 pour le passage du sang à l'intérieur du manchon.

Comme on le comprend, il est possible, en jouent sur les dimensions du ballon 2 (notamment de la largeur de la cavité interne 10), d'obtenir une large lumière 13.

Le manchon 11 est relié à son extrémité arrière, par l'intermédiaire d'une paroi conique 14 à un tube coulissant 15 de section transversale sensiblement circulaire, dont le diamètre interne est légèrement supérieur au diamètre externe de l'élément tubulaire 1 et dont le diamètre externe est inférieur au diamètre interne du tube 5. Le tube 15 est par conséquent déplaçable de façon guidée le long de l'élément tubulaire 1 formant ainsi un moyen de commande en déplacement longitudinal du manchon 11.

La paroi conique 14 comporte un ou plusieurs orifices 16 permettant le passage à l'intérieur du manchon 11 du sang provenant du vaisseau sanguin à travers les orifices 3 situés en amont du ballon 2 (voir flèches F).

De préférence, le manchon 11 comporte à son extrémité avant une paroi conique 17 destinée à faciliter la mise en place du manchon 11 dans sa position de travail représentée à la figure 3.

La paroi conique avant 17 comporte un ou plusieurs orifices 18 coopérant avec les orifices 4 situés en aval du ballon 2 pour assurer le passage du sang de l'intérieur du manchon 11 vers la partie du vaisseau sanguin situé en aval du ballon (voir flèches F₁).

Le manchon 11 pourra être réalisé d'une seule pièce avec la paroi conique avant 17, la paroi conique arrière 14 et le tube 15 par exemple en une matière telle que celle servant à réaliser le tube 5 ou bien encore en un fluor éthylène propylène (F.E.P.) qui présente de bonnes caractéristiques de friction.

Sur les figures 1 à 3, les chiffres de référence 20, 21, 22, 23 désignent des orifices respectivement prévus dans l'élément tubulaire interne 1, dans la paroi avant 17 du manchon 11, dans la paroi arrière 16 du manchon 11 et dans la partie arrière de la gaine 6, pour le passage latéral d'un guide.

Les orifices 22 et 23 se trouvent sensiblement en vis-à-vis lorsque le manchon 11 se trouve en position de repos représentée à la figure 2.

Le chiffre de référence 24 désigne un élément métallique radio-opaque dont la fonction est de permettre de visualiser la position du ballon 2 par radioscopie. Il s'agit d'un élément largement décrit dans l'état de la technique. Dans l'exemple représenté, cet élément est réalisé sous forme d'une bague disposée autour de l'élément tubulaire interne 1.

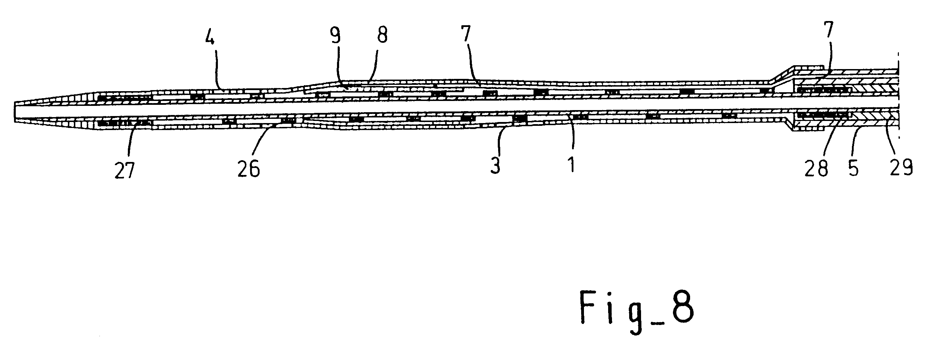

On a représenté aux figures 8 à 10 la partie distale d'un cathéter de dilatation conforme à un deuxième mode de réalisation de l'invention.

Les éléments identiques à ceux décrits en référence au premier mode de réalisation sont désignés par des chiffres de référence identiques.

Dans ce deuxième mode de réalisation de l'invention, l'élément de support précité comprend un ressort hélicoïdal 26 dont l'extrémité avant 27 disposée en aval du ballon 2 est fixe, par exemple soudé à l'élément tubulaire interne 1, et dont l'extrémité arrière 28 est mobile, par exemple en étant fixé dans un logement prévu dans un tube coulissant 29 semblable eu tube 15 décrit en référence au premier mode de réalisation.

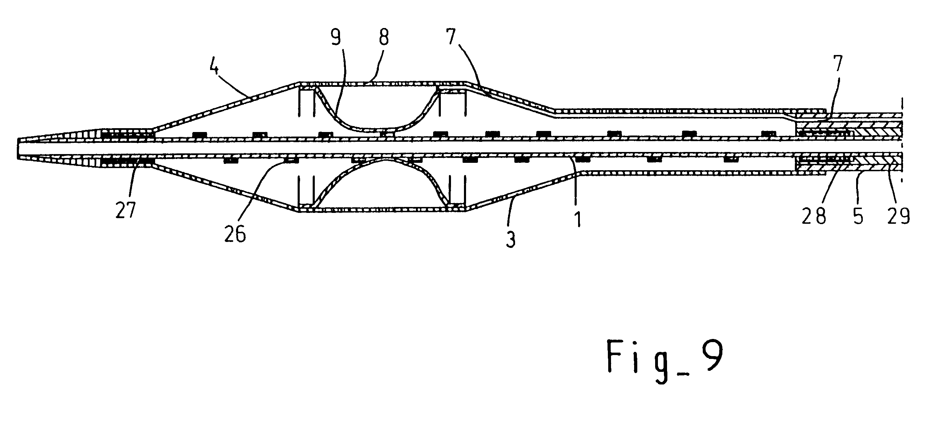

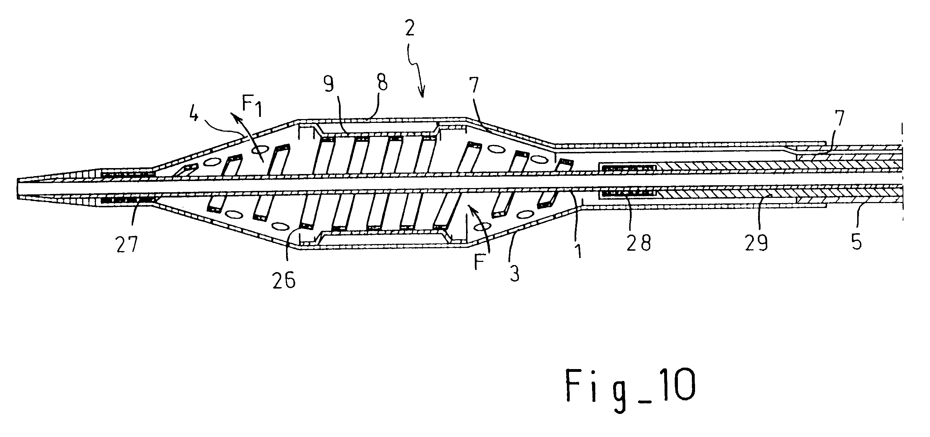

Le ressort 28 est susceptible d'occuper une position d'allongement, représentée aux figures 8 et 9, obtenue par traction de l'extrémité arrière 28, et une position de repos représentée à la figure 10, dans laquelle ses spires situées au niveau du ballon viennent supporter et maintenir la paroi interne 9 du ballon, en l'écartant de l'élément tubulaire 1 et en créent ainsi une large lumière pour le passage du sang.

Le diamètre externe des spires du ressort 26 en position de repos est supérieur au diamètre externe de ces spires en position d'allongement.

Le flux sanguin peut donc traverser l'extrémité du cathéter de dilatation en entrant par les orifices 3 et en sortant par les orifices 4.

Pour obtenir la position d'allongement précitée, il suffira donc d'exercer une traction sur le tube coulissant 29.

Le ressort 26 pourra être réalisé par exemple en un fil de préférence de section transversale rectangulaire, en acier inoxydable, par exemple norme A.I.S.I. 304, ou en alliage métallique à mémoire de forme et haute élasticité, par exemple du type NITINOL ®.

Ce ressort hélicoïdal peut être éventuellement remplacé par toute structure déformable radialement par rapprochement de ses extrémités comme une grille ou une tresse métallique.

Les cathéters conformes aux deux modes de réalisation qui viennent d'être décrits pourront être utilisés relativement aisément, par une technique très proche de celle décrite dans l'état de la technique.

Ainsi, le cathéter sera introduit à l'aide d'un guide métallique d'une façon classique, jusqu'au niveau de la sténose. Lors de cette opération, la gaine externe 6 sera à l'état replié représenté aux figures 1 et 8. Les dimensions du cathéter sont alors tout à fait comparables à celles d'un cathéter de dilatation classique.

Le ballon 2 est ensuite gonflé pour dilater la sténose d'une façon classique comme représenté aux figures 2 et 9.

Le ballon 2 est ensuite dégonflé pour permettre la mise en place du manchon 11 ou du ressort 26.

Puis le ballon 2 est gonflé une deuxième fois pour dilater à nouveau la sténose tout en permettant le passage quasiment normal du flux sanguin au travers du cathéter (figures 3 et 10).

Le cathéter peut rester dans cette dernière position pendant un temps très long ( de l'ordre de plusieurs dizaines de minutes, si cela est nécessaire) et permettre ainsi un traitement durable de la sténose.

## Revendications

1. Cathéter de dilatation, destiné à être introduit dans un canal corporel tel que notamment un vaisseau sanguin, comprenant :
- un élément tubulaire externe comportent une partie distale et une partie proximale, présentent au niveau de sa partie distale une portion déformable radialement (6) comportant un ballon (2), et de part et d'autre de ce ballon au moins un orifice (3, 4) communiquant avec ledit canal corporel et permettent le passage du fluide circulent dans le canal corporel à l'intérieur dudit élément tubulaire externe ;
- un élément tubulaire interne (1) disposé intérieurement audit élément tubulaire externe, et permettant le passage d'un guide;
- un micro-tube (7), disposé de préférence intérieurement audit organe tubulaire externe, débouchant à une extrémité à l'intérieur dudit ballon et relié à son autre extrémité à une source d'alimentation en fluide pour permettre le gonflage et le dégonflage dudit ballon;
- ledit ballon (2) comprenant une paroi externe (8) destinée à venir en contact avec le paroi interne dudit canal corporel, en position d'utilisation caractérisé en ce que : une paroi interne (9) est fixée de manière étanche à ladite paroi externe et est susceptible de délimiter avec celle-ci, en position d'utilisation, une cavité interne (10) annulaire ;
- et en ce qu'il comprend en outre un élément de support conformé pour maintenir en position d'utilisation ladite paroi interne du ballon, tout en permettant la circulation du fluide (1) corporel dans l'espace compris entre ledit élément interne et la paroi interne (9) du ballon.

2. Cathéter de dilatation selon le revendication 1, caractérisé en ce que l'élément tubulaire externe comprend un tube flexible 5 sur la partie avant duquel est fixée de façon étanche une partie formant gaine (6) déformable radialement et faisant corps dans le ballon (2).

3. Cathéter de dilatation selon la revendication 1 ou 2, caractérisé en ce que l'élément de support précité comprend un manchon (11) de forme généralement cylindrique, déplaçable longitudinalement entre une position de repos, dans laquelle il se trouve en amont du ballon (2) et une position de travail dans laquelle il vient supporter et maintenir la paroi interne (9) du ballon (2) en l'écartant de l'élément tubulaire (1) et en créant ainsi une lumière (13) pour le passage du fluide corporel à l'intérieur du manchon.

4. Cathéter de dilatation selon la revendication 3, caractérisé en ce que le manchon (11) est relié à son extrémité arrière, par l'intermédiaire d'une paroi conique (14) à un tube coulissant (15), de section transversale sensiblement circulaire, dont le diamètre interne est légèrement supérieur au diamètre externe de l'élément tubulaire (1) et dont le diamètre externe est inférieur au diamètre interne du tube (5) précité.

5. Cathéter de dilatation selon la revendication 4, caractérisé en ce que la paroi conique (14) précitée comporte un ou plusieurs orifices (16) permettant le passage à l'intérieur du manchon (11) du fluide corporel précité.

6. Cathéter de dilatation selon l'une des revendications 3 à 5, caractérisé en ce que le manchon (11) précité comporte à son extrémité avant une paroi conique (17) comportant un ou plusieurs orifices (18) assurant le passage du fluide corporel de l'intérieur du manchon (11) vers la partie du canal corporel située en aval du ballon (2).

7. Cathéter de dilatation selon la revendication 1 ou 2, caractérisé en ce que l'élément de support précité comprend un ressort hélicoïdal (26) dont l'extrémité avant (27) disposée en aval du ballon (2) est fixe, et dont l'extrémité arrière (28) est mobile ; ledit ressort (28) étant susceptible d'occuper une position d'allongement, obtenue par traction de son extrémité arrière (28) et une position de repos dans laquelle ses spires situées au niveau du ballon (2) viennent supporter et maintenir la paroi interne (9) du ballon, en l'écartant de l'élément tubulaire (1) et en créant ainsi une lumière pour le passage du fluide corporel.

8. Cathéter de dilatation selon la revendication 7, caractérisé en ce que l'extrémité arrière (28) du ressort (26) précité est fixée dans un logement prévu dans un tube coulissant (29) dont le diamètre interne est légèrement supérieur au diamètre externe de l'élément tubulaire (1) et dont le diamètre externe est inférieur au diamètre interne du tube (5) précité.

## Claims

1. Dilating catheter, intended to be introduced in a bodily duct such as in particular a blood vessel, comprising:
- an outer tubular element comprising a distal part and a proximal part, presenting at the level of its distal part a radially deformable portion (6) comprising a balloon (2), and on either side of this balloon at least one orifice (3, 4) communicating with said bodily duct and allowing the passage of the fluid circulating in the bodily duct within said outer tubular element,
- an inner tubular element (1) disposed inside said outer tubular element and allowing the passage of a guide;
- a micro-tube (7), preferably disposed inside said outer tubular element, opening out at one end inside said balloon and connected at its other end to a source of fluid supply to allow inflation and deflation of said balloon;
- said balloon (2) comprising an outer wall (8) intended to come into contact with the inner wall of said bodily duct, in position of use, characterized in that:
- an inner wall (9) is hermetically fixed to said outer wall and is capable of defining therewith, in position of use, an annular inner cavity (10);
- and in that it further comprises a support element shaped in order to maintain said inner wall of the balloon in position of use, while allowing circulation of the bodily fluid (1) in the space included between said inner element and the inner wall (9) of the balloon.

2. Dilating catheter according to Claim 1, characterized in that the outer tubular element comprises a flexible tube 5 on the front part of which is hermetically fixed a radially deformable sheath-forming part (6) forming part in the balloon (2).

3. Dilating catheter according to Claim 1 or 2, characterized in that the said support element comprises a sleeve (11) of generally cylindrical form, displaceable longitudinally between a position of rest in which it lies upstream of the balloon (2) and a working position in which it supports and maintains the inner wall (9) of the balloon (2), spacing it apart from the tubular element (1) and thus creating a slot (13) for the passage of the bodily fluid inside the sleeve.

4. Dilating catheter according to Claim 3, characterized in that the sleeve (11) is connected at its rear end, via a conical wall (14), to a sliding tube (15), of substantially circular transverse section, whose inner diameter is slightly greater than the outer diameter of the tubular element (1) and whose outer diameter is less than the inner diameter of said tube (5).

5. Dilating catheter according to Claim 4, characterized in that said conical wall (14) comprises one or more orifices (16) allowing passage inside the sleeve (11) of said bodily fluid.

6. Dilating catheter according to one of Claims 3 to 5, characterized in that said sleeve (11) comprises at its front end a conical wall (17) comprising one or more orifices (18) ensuring passage of the bodily fluid inside the sleeve (11) towards that part of the bodily duct located downstream of the balloon (2).

7. Dilating catheter according to Claim 1 or 2, characterized in that said support element comprises a helical spring (26) of which the front end (27) disposed downstream of the balloon (2) is fixed and of which the rear end (28) is mobile; said spring (28) being capable of occupying a position of elongation, obtained by traction of its rear end (28) and a position of rest in which its turns located at the level of the balloon (2) support and maintain the inner wall (9) of the balloon, spacing it apart from the tubular element (1) and thus creating a slot for the passage of the bodily fluid.

8. Dilating catheter according to Claim 7, characterized in that the rear end (28) of said spring (26) is fixed in a housing provided in a sliding tube (29) whose inner diameter is slightly greater than the outer diameter of the tubular element (1) and whose outer diameter is less than the inner diameter of said tube (5).

## Patentansprüche

1. Dilatationskatheter zum Einführen in einen Körperkanal wie insbesondere ein Blutgefäß, umfassend
- einen röhrchenförmigen Außenbestandteil, welcher einen entfernten Bereich und einen nahen Bereich umfaßt und in seinem entfernten Bereich einen radial verformbaren Teil (6) besitzt, der einen Schlauch (2) und auf dessen beiden Seiten mindestens eine Öffnung (3, 4) enthält, die mit dem Körperkanal in Verbindung steht und den Durchgang der Flüssigkeit, die in diesem Körperkanal zirkuliert, ins Innere des röhrchenförmigen Außenbestandteils ermöglicht,
- einen in dem röhrchenförmigen Außenbestandteil angeordneten röhrchenförmigen Innenbestandteil (1), der den Durchgang einer Führung erlaubt, und
- eine vorzugsweise in dein röhrchenförmigen Außenbestandteil angeordnete Mikroleitung (7), deren eines Ende am Inneren des Schlauchs mündet und deren anderes Ende mit einer Fluidversorgung verbunden ist, um das Ausdehnen und Zusammenziehen dieses Schlauchs zu ermöglichen,
wobei der Schlauch (2) eine Außenwand (8) enthält, die vorgesehen ist, in Einsatzposition mit der Innenwand des Körperkanals in Berührung zu kommen, **dadurch gekennzeichnet, daß** an der Außenwand eine Innenwand (9) dicht befestigt ist, die in Einsatzposition mit jener einen ringförmigen Innenhohlraum (10) begrenzen kann **und daß** er außerdem ein Tragelement umfaßt, das ausgebildet ist, um in Einsatzposition die Innenwand des Schlauchs zu halten, wobei die Zirkulation der Körperflüssigkeit in den Zwischenraum zwischen dem röhrchenförmigen Innenbestandteil (1) und der Innenwand (9) des Schlauchs aufrechterhalten bleibt.

2. Dilatationskatheter nach Anspruch 1, **dadurch gekennzeichnet,** daß der röhrchenförmige Außenbestandteil einen flexiblen Schlauch (5) umfaßt, auf dessen vorderen, Teil ein Teil dicht befestigt ist, das eine Hülle (6) bildet, die radial verformbar und mit dein Schlauch (2) fest verbunden ist.

3. Dilatationskatheter nach Anspruch 1 oder 2, **dadurch gekennzeichnet, daß** das Tragelement einen im allgemeinen zylindrischen Mantel (11) umfaßt, der zwischen einer Ruheposition, in welcher er sich in Strömungsrichtung vor dem Schlauch (2) befindet, und einer Arbeitsposifion längsbeweglich ist, in welcher er die Innenwand (9) des Schlauchs (2) trägt und hält, wobei er sie von dem röhrchenförmigen Innenbestandteil (1) beabstandet und so eine Öffnung (13) für den Durchgang der Körperflüssigkeit in das Innere des Mantels schafft.

4. Dilatationskatheter nach Anspruch 3, **dadurch gekennzeichnet, daß** der Mantel (11) mit seinem hinteren Ende über eine sich verjüngende Wand (14) mit einem hin- und hergehenden Schlauch (15) verbunden ist, dessen Querschnitt im wesentlichen kreisrund, dessen Innendurchmesser etwas größer als der Außendurchmesser des röhrchenförmigen Innenbestand-teils (1) und dessen Außendurchmesser kleiner als der Innendurchmesser des flexiblen Schlauchs (5) ist.

5. Dilatationskatheter nach Anspruch 4, **dadurch gekennzeichnet, daß** die sich verjüngende Wand (14) eine oder mehrere Öffnun-gen (16) enthalt, die den Durchgang der Körperflüssigkeit in das Innere des Mantels (11) erlauben.

6. Dilatationskatheter nach einem der Ansprüche 3 bis 5, **dadurch gekennzeichnet, daß** der Mantel (11) in seinem vorderen Ende eine sich vezjüngende Wand (17) umfaßt, die eine oder mehrere Öffnungen (18) enthalt, die den Durchgang der Körperflüssigkeit vom Inneren des Mantels (11) zu dem Teil des Körperkanals sicherstellen, der sich hinter dem Schlauch (2) befindet.

7. Dilatationskatheter nach Anspruch 1 oder 2, **dadurch gekennzeichnet, daß** das Tragelement eine zylindrische Schraubenfeder (26) umfaßt, deren vorderes, nach dem Schlauch (2) angeordnetes Ende (27) fest und deren hinteres Ende (28) beweglich ist, wobei die Feder (28) eine Dehnungsposition, die durch Zug auf ihr hinteres Ende (28) erhalten wird, und eine Ruheposition einnehmen kann, in welcher ihre im Schlauch (2) befindlichen Windungen die Innenwand (9) des Schlauchs tragen und halten, wobei sie sie von dem röhrchenförmigen Innenbestandteil (1) beabstanden und so eine Öffnung für den Durchgang der Körperflüssigkeit schaffen.

8. Dilatationskatheter nach Anspruch 7, **dadurch gekennzeichnet, daß** das hintere Ende (28) der Feder (26) in einer Aufnahme befestigt ist, die in einem hin- und hergehenden Schlauch (29) vorgesehen ist, dessen Innendurchmesser etwas größer als der Außendurchmesser des röhrchenförmigen Innenbestandteils (1) und dessen Außendurchmesser kleiner als der Innendurchmesser des flexiblen Schlauchs (5) ist.
